Europäisches Patentamt

European Patent Office

Office européen des brevets

(19)

(11) Veröffentlichungsnummer: **0 164 595**
A1

## (12) EUROPÄISCHE PATENTANMELDUNG

(21) Anmeldenummer: **85105898.2**

(22) Anmeldetag: **14.05.85**

(51) Int. Cl.⁴: **A 61 F 13/16**
**A 61 F 13/18**

(30) Priorität: **15.05.84 DE 3417909**

(43) Veröffentlichungstag der Anmeldung:
**18.12.85 Patentblatt 85/51**

(84) Benannte Vertragsstaaten:
**AT BE CH DE FR GB IT LI LU NL SE**

(71) Anmelder: **Dobson, Brigitte**
**Seeadlerring 3**
**D-3004 Isernhagen 1(DE)**

(72) Erfinder: **Dobson, Brigitte**
**Seeadlerring 3**
**D-3004 Isernhagen 1(DE)**

(54) **Neuartige hygienische Vorlage.**

(57) Beschrieben wird eine neuartige, hygienische Vorlage, die eine der Vulva angepasste Vertiefung aufweist.

EP 0 164 595 A1

Croydon Printing Company Ltd.

## Neuartige hygienische Vorlage

Die Erfindung betrifft eine hygienische Vorlage, die die Ausscheidungen und Sekretionen beispielsweise während der Menstruation auffängt.

Als Vorlagen sind beispielsweise Damenbinden und Slipeinlagen bekannt, die zum Schutz der Kleidung die Menstruationsblutungen und andere Sekretionen auffangen. Diese Vorlagen weisen verschiedene Stärken, unterschiedliche Halterungen und unterschiedliches Resorptionsvermögen auf. Hauptsächlich sind rechteckige Formen erhältlich, dessen Längsachse ungefähr der Länge und dessen Breitenachse ungefähr der breitesten Stelle des weiblichen Perineums entspricht.

Entscheidend für die Schutzwirkung ist eine exakte und dauerhafte Fixierung dieser Vorlagen. Lösungsvorschläge finden sich in Form von Haltegürteln, speziell gestalteten Monatshöschen oder neuerdings in Binden, die mit Klebestreifen zur Befestigung im Slip versehen sind.

All diese Vorlagen weisen jedoch Nachteile auf: sie sind entweder unbequem oder unter enger Kleidung nicht unbemerkt zu tragen und bieten nur einen mehr oder weniger genügenden Schutz der Kleidung vor Verschmutzung. Die Gründe hierfür liegen darin, daß die Binden trotz aller Fixierungsversuche leicht verrutschen oder beim Gehen, durch Veränderung der Körperstellung und durch Anpassung an den Körper meist automatisch zusammengedrückt werden oder so verrutschen, daß die Ausscheidungen an den Seiten vorbeifließen können.

Es sind zwar Flachbinden beschrieben, die der Körperform angepaßt sind und eine Mittelrinne aufweisen (s. DE-PS 11 70 581), und als Weiterentwicklung dieser zur Erhöhung des Flüssigkeitsaufnahmevermögen und zur besseren Abdichtung einen am hinteren Ende der Mittelrinne des Saugkörpers befindlichen Abschluß-Höcker zeigen (s. DE-PS 31 36 014), doch besteht auch bei diesen Binden die Gefahr der "Deformation" durch Körperbewegungen, so daß auch sie das Vorbeifließen nicht zuverlässig verhindern können.

Bei allen bekannten Binden besteht das Problem darin, daß sich zwischen der Vulva und der Binde stets ein Zwischenraum befindet. Die Binde kommt dadurch mit dem M. gluteus maximus in einem Bereich in Berührung, der bei Veränderungen der Körperstellung besonders stark bewegt wird und dadurch auch die Binde zusammendrückt und verschiebt.

Zur Vermeidung der Wäscheverschmutzung sind die meisten bekannten Binden mit einer undurchlässigen Folie ausgestattet, die aus nicht wasserlöslichen Materialien, meistens aus Polyethylen hergestellt sind. Diese Materialien erschweren jedoch eine hygienische Beseitigung.

Aufgabe der vorliegenden Erfindung war es daher, eine Vorlage zu entwickeln, die
- a) durch die beim Gehen oder durch Veränderung der Körperstellung auftretenden Verformungen ihre Funktionsfähigkeit nicht einbüßt,
- b) bequemes Tragen ermöglicht und
- c) hygienisch und umweltfreundlich beseitigt werden kann.

Erfindungsgemäß wird diese Aufgabe dadurch gelöst, daß eine in an sich bekannter Weise hergestellte Vorlage eine der Vulva angepaßte Vertiefung aufweist.

Diese rinnenförmige Vertiefung in der Vorlage nimmt die Vulva auf (Abb.I-IV.3). Die erhöhten, die Vulva einschließenden Ränder verhindern ein seitliches Vorbeifließen der Ausscheidungen. Eine bevorzugte Ausführung sieht im vorderen und hinteren Teil der Vorlage ebenfalls einen begrenzenden, erhöhten Rand vor, der ein Vorbeifließen der Ausscheidungen nach vorne bzw. hinten verhindert, eine Ausführung, die insbesondere in Ruhepositionen vorteilhaft ist (Abb.I und II,2 bzw. 4). Eine weitere Ausführung der Vorlage sieht eine muldenartige, der Vulva angepaßte Form der Vertiefung vor (Abb.III und IV).

Die die Vertiefung begrenzenden Ränder aus resorbierenden Materialien können einen rechteckigen, runden oder elliptischen Querschnitt zeigen. Weiterhin können die seitlichen Ränder mit dem übrigen resorbierenden Material vollflächig oder vorzugsweise nur an den äußeren Grenzen der Vorlage befestigt sein. Die Befestigung nur an den äußeren Grenzen ermöglicht ein "Aufklappen" der Ränder. Diese flexible, nachgiebige Ausgestaltung gewährleistet noch mehr Sicherheit bei Bewegungen. Das resorbierende Material kann gegebenenfalls mit einem nicht resorbierenden Gewebe umgeben werden.

Um Interferenzen mit dem Innenschenkel und dem M. gluteus maximus zu vermeiden, sind die lateralen Seiten im hinteren Teil der Vorlage konkav weggeschnitten (Abb.I und III). Eine schmale, gegebenenfalls weiche Verlängerung der nicht resorbierenden Unterschicht der Vorlage im hinteren Bereich paßt sich dem glutealen Spalt an, und bietet dadurch eine noch bessere Fixierung der Vorlage an der Vulva und ermöglicht ein sicheres und bequemes Tragen (Abb.I-IV,5). Aus dem gleichen Grund kann auch der vordere Bereich der nicht resorbierenden Unterschicht der Vorlage eine Verlängerung aufweisen (Abb.I-IV,1).

Zur Herstellung der erfindungsgemäßen Vorlagen kommt jedes handelsübliche, für hygienische Binden verwendbare Material in Frage. Dabei kann durch die der Vulva angepaßte Form der Vertiefung und die anatomisch angepaßte Form der Vorlage selbst der resorbierende Teil der Vorlage auf ein zweckmäßiges Minimum beschränkt werden. Geformt werden kann die
erfindungsgemäße Vorlage prinzipiell mit den Maschinen, die
für handelsübliche Binden Verwendung finden, nachdem sie
entsprechend den erfindungsgemäßen Ausgestaltungsmerkmalen
abgewandelt worden sind.

Zur sicheren Fixierung kann das Unterteil der Vorlage
vollständig oder auch nur teilweise mit Klebestreifen versehen
werden, die eine Befestigung im Slip ermöglichen (Abb.II und
I V, 7).

Wie bereits erwähnt, beinhalten die meisten bisher bekannten
Vorlagen im Bereich der resorbierenden Schicht einen nicht
wasserlöslichen Kunststoffilm, der ein Durchdringen der
Ausscheidungen zur Kleidung vermeiden soll. Durch diesen Film
ist jedoch eine hygienische Vernichtung, beispielsweise durch
Wegspülen nicht möglich. Bei der erfindungsgemäßen Vorlage
besteht dieser Film aus wasserlöslichen, für die
Ausscheidungen undurchdringlichen Materialien, vorzugsweise
aus Polyvinylalkohol, die eine einfache, hygienische und
umweltfreundliche Beseitigung ermöglichen.

Die gezeigten Abbildungen geben zwei mögliche Ausführungen im
Maßstab von ca. 1:2 wieder, ohne die Erfindung dadurch in
irgendeiner Weise beschränken zu wollen.

Abb.I und III: Aufsicht

Abb. II und IV: Seitenansicht mit Schnitt durch die
Längsachse. Die gebogenen Pfeile 6 weisen darauf hin, daß die
lateralen Ränder zur Seite weggeklappt werden können.

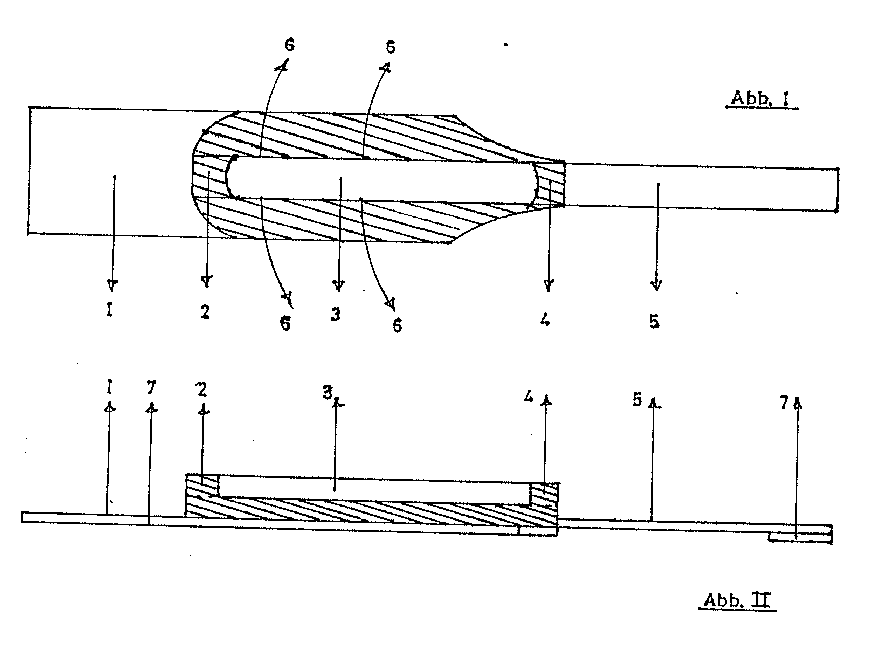

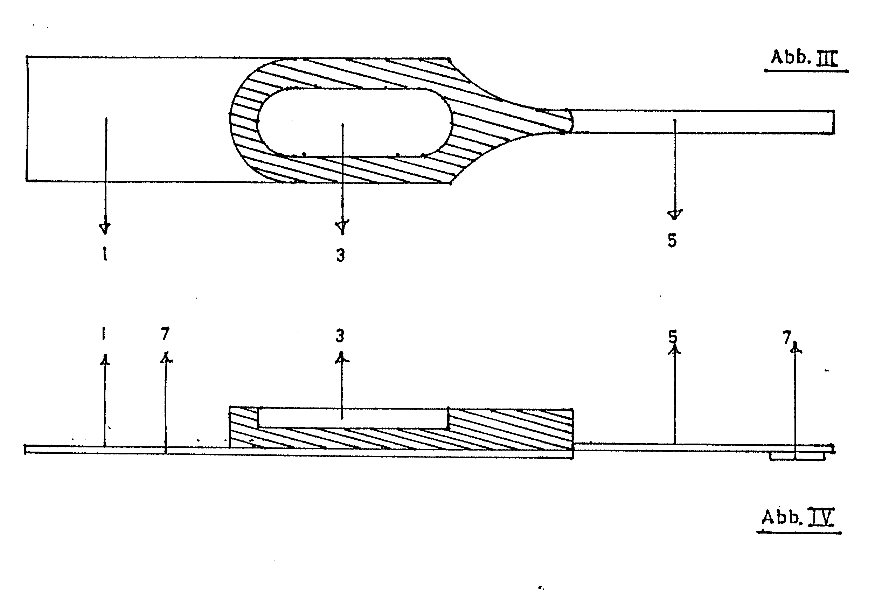

# PATENTANSPRÜCHE

1) Vorlage, die in an sich bekannter Weise aus einem resorbierenden Material und einem dieses umgebenden nicht resorbierenden Gewebe besteht, dadurch gekennzeichnet, daß sie eine rinnenförmige Vertiefung aufweist, die in Form und Abmessungen der Vulva angepaßt ist.

2) Vorlage nach Anspruch 1, dadurch gekennzeichnet, daß die rinnenförmige Vertiefung im vorderen und hinteren Teil einen begrenzenden, erhöhten Rand aufweist, der aus resorbierendem Material besteht.

3) Vorlage nach einem der vorhergehenden Ansprüche, dadurch gekennzeichnet, daß die rinnenförmige Vertiefung eine muldenartige Form aufweist.

4) Vorlage nach einem der vorhergehenden Ansprüche, dadurch gekennzeichnet, daß die die rinnenförmige Vertiefung begrenzenden Ränder einen rechteckigen, runden oder elliptischen Querschnitt aufweisen.

5) Vorlage nach einem der vorhergehenden Ansprüche, dadurch gekennzeichnet, daß die die rinnenförmige Vertiefung begrenzenden lateralen Ränder nur an den äußeren Grenzen der Vorlage befestigt sind, so daß ein "Aufklappen" möglich ist.

6) Vorlage nach einem der vorhergehenden Ansprüche, dadurch gekennzeichnet, daß die lateralen Seiten im hinteren Teil der Vorlage einen konkav geformten Ausschnitt aufweisen.

7) Vorlage nach einem der vorhergehenden Ansprüche, dadurch gekennzeichnet, daß die Vorlage im hinteren Bereich konkav verjüngt ist und die nicht resorbierende Unterschicht eine schmale, gegebenenfalls weiche Verlängerung aufweist.

8) Vorlage nach einem der vorhergehenden Ansprüche, dadurch gekennzeichnet, daß die nicht resorbierende Unterschicht der Vorlage im vorderen Bereich eine Verlängerung aufweist.

9) Vorlage nach einem der vorhergehenden Ansprüche, dadurch gekennzeichnet, daß in das resorbierende Material ein kleiderschützender, wasserlöslicher Film eingearbeitet ist.

10) Vorlage nach einem der vorhergehenden Ansprüche, dadurch gekennzeichnet, daß der wasserlösliche Film aus Polyvinylalkohol hergestellt ist.

11) Vorlage nach einem der vorhergehenden Ansprüche, dadurch gekennzeichnet, daß sie an der Unterschicht ganz oder teilweise mit Klebestreifen versehen ist.

Abb. I

Abb. II

½

0164595

Abb. III

1        3        5

1   7     3     5   7

Abb. IV

2/2

0164595

## EINSCHLÄGIGE DOKUMENTE

EP 85105898.2

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | DE - A1 - 2 718 344 (FRANCO) <br> * Ansprüche 1,7; Seite 9, Zeilen 30-33; Fig. 1,2 * <br> -- | 1-4,11 | A 61 F 13/16 <br> A 61 F 13/18 |
| X | FR - A1 - 2 420 339 (BEGHIN-SAY) <br> * Ansprüche 1-3; Fig. 1,2 * <br> -- | 1-4 | |
| A | DE - A1 - 3 042 322 (HENKEL) <br> * Fig. 1 * <br> -- | 5 | |
| A | CH - A - 453 573 (RIEGEL) <br> * Fig. 1,3 * <br> -- | 7,8 | |
| A | DE - A1 - 3 015 538 (ZENMI) <br> * Anspruch 1 * <br> ---- | 9,10 | |
| | | | RECHERCHIERTE SACHGEBIETE (Int Cl 4) <br><br> A 61 F |

Der vorliegende Recherchenbericht wurde für alle Patentansprüche erstellt

| Recherchenort | Abschlußdatum der Recherche | Prüfer |
|---|---|---|
| WIEN | 21-08-1985 | FARNIOK |